# EUROPEAN PATENT APPLICATION

(11) **EP 2 014 224 A1**
(43) Date of publication of application: **14.01.2009**
(21) Application number: 08016940.2
(22) Date of filing: 24.03.2006
(51) Int. Cl.: A61B 5/00, G06F 19/00

(54) **Telemedical method and device for the calibration of measured values**

(62) Divisional of application: 06006190.0
(71) Applicant: GlucoTel Scientific Inc., Reno NV 89511 (US)
(72) Inventor: Sickmann, Jan, 35104 Lichtenfeld (DE)
(74) Representative: Reinhardt, Thomas Johannes

(57) **Abstract**

Method and apparatus for processing measured values being related to a human body, in which a property of human substance is measured by a measuring device (30) sensing characteristic changes of a test device (41) effectuated when having contact with said human substance, wherein one or more electrical quantities are obtained from said measurement, which are converted into digital measured values, further evaluated at a communication device (20) communicating with said measurement device (30) wherein said communication device (20) making a plausibility check on time related data associated with a data set generated by said measurement device (30).

## Description

### 1. BACKGROUND OF THE INVENTION

### 1.1. FIELD OF THE INVENTION

The present invention is in the field of telemedicine systems and relates in particular to a system of improved operability More particularly, it relates to a method and system for processing measured values being related to a human body, wherein a property of a human substance i.e. blood, urine, saliva, etc. is measured by a measuring instrument by sensing characteristic changes of a test device, e.g. a test strip as known from glucose measurements, wherein the changes are effected when having contact with the human substance, and wherein one or more electrical quantities, e.g., voltage, current, capacity, are obtained from the measurement.

### 1.2. DESCRIPTION AND DISADVANTAGES OF PRIOR ART

Although the present invention can be applied to a variety of different applications, it will be set forth and defined from prior art in its special reference to the measurements of glucose in human blood as it is required for diabetes mellitus patients.

In the year of 2000, more than 151 million people in the world were diabetic. It is predicted that by 2010, 221 million people and by 2025, a number of 324 million will be diabetic. There are two major forms of diabetes: type 1 and type 2 diabetes. The hall mark of type 1 diabetes is the destruction of insulin producing β-cells in the pancreas, primarily due to autoimmune responses. Type 1 diabetes is manifested with absolute insulin deficiency. In contrast, type 2 diabetes is characterized by two defects: insulin deficiency and insulin resistance. Type 2 diabetes accounts for 90 to 95% of the incidence of diabetes. The current epidemic outbreak of diabetes reflects the high prevalence of type 2 diabetes.

Type-1 diabetes patients usually must measure regularly (several times a day) their glucose level in the blood, for type-2 diabetics this is at least strongly recommended to do it regularly in larger intervals of time. A precise glucose level measurement is key for the appropriate, individual medical care. Thus glucose measurement is a very important concern for many millions of people.

In order to determine the current glucose level of a diabetic person, prior art primarily uses biochemical reactions. More particularly, in a certain type of prior art the chemical substance glucose reacts with specific other chemical reactance provided on the test strip (for example glucose-specific enzymes like glucose-dehydrogenase). As a consequence, reaction products are generated, of which the quantity or colour intensity on the test strip is proportional to the quantity of glucose in the blood sample in use. These prior art methods sense those reaction products by aid of a built-in photometer responsive to colour intensity or by aid of measuring electrodes responsive to electrical charge within the device.

Further prior art methods rely on other physical properties of the blood, for example its viscosity. Such methods however, are not yet broadly accepted. A person skilled in the art will appreciate that the metering methods relying either on colour intensity or on chemical charge are quite selective for sensing the glucose within the blood, but are disadvantageously concurrently error-prone due to the influence of external margin conditions like extreme temperature, or due to small quantities of substances being accidentally deposited on the surface of the skin and being involved during pricking the skin, or impurities on the test strip. Further, a too small sample quantity or other influences, for example nutrition-specific influences may render the measured data un-precise. Thus, a diabetic is basically confronted with a significant burden when being confronted with its daily task of measuring his glucose level.

Further, type-I diabetics need to monitor their blood glucose levels regularly. This requires typically a small blood sample to be obtained by pricking the skin, usually on a finger and placing the sample on a test strip which is read by an electronic glucose meter. In this system, the glucose meter and the test strip are strongly recommended to be provided by the same producer. An example of such prior art glucose meter system is the system "major II", commercially available by Medpro GmbH, 23923 Lüdersdorf, Germany.

Self-monitoring in this way helps to detect when blood sugar levels may be too low or too high. Both situations may threaten the patient's health. Usually, diabetics should carefully record any relevant event in a patient diary. Those events comprise body weight, current urinal and blood glucose values as well as unusual events like eating an unusual quantity of sugar in form of cakes, chocolate, etc. or periods of extreme body work, etc.. This is a burdensome task.

Prior art telemedicine systems as for example published in WO 2004/027676A3 or in DE 10140968A1, or in DE 101 02 564 A1 try to facilitate this burden by offering a telemedicine subsystem comprising a smartphone (for example a PDA) in connection and operable with an electronic glucose meter and an associated test strip according to the preamble of an independent claim appended herein. Also, the above-mentioned MAJOR II system belongs to such prior art.

A general problem for all those being confronted with measuring blood glucose levels is that the glucose level can not be directly derived from the sensing unit itself, but instead an electrical value (e.g., current, voltage, capacity) obtained within the measurement must be converted into a digital value which can be interpreted as a glucose value only after being further converted by a procedure, which is commonly referred to in this field of the art as "calibration".

Although this term might be used scientifically in a different sense, namely to make disappear a difference between a displayed measuring value and the "true" measuring value by adjusting the metering device, the terms "calibration", to "calibrate" are used in this document for transforming a measured value into an application-driven directly interpretable value. For example, a measured value of 37 mA will be transformed into a glucose level of 130 dimensioned according to the commonly applied rules in the medical art. Or, a measured voltage of 3,1 Volt is transformed by the "calibration" procedure to a weight of 58,5 kg, if the electrical sensor outputting this voltage would be used in a scales device, improved by the present invention. Thus, in many use cases, one or more characteristic curves covering some permitted measurable range, maybe accompanied by some time information or other information, e.g., temperature, is herein understood as "calibrating information". This defines the mathematical base for a calibration rule usable for transforming the measured values into a usable, directly understandable value.

If a given glucose measurement is calibrated with a wrong calibration rule the resulting glucose level is quite unprecise, which in turn may lead the diabetes patient to consume a too large or a too small quantity of insulin, or sugar-containing nutrition, respectively. Both types of deviations can threaten the patient's health. Thus, it is a key interest for a diabetes patient to know exactly about his current glucose level.

The before-mentioned patent document WO 2004/027676 does not really care of this special calibration problem: A combined electronic physiological data acquisition unit is proposed therein for measuring one or more physiological parameters - for example the glucose level - of a patient to acquire and output data representing the parameter and a wireless transmitter which upon receiving the output data from the data acquisition unit automatically transmits the output data via a wireless network to a remote server, where that data is further evaluated. No particular disclosure is provided for the specific problem how the measured data is calibrated and how this can be done also in emergency situations. It is just described that "the device may be adapted to check the acquired data for compliance with preset conditions, such as concerning the quality of completeness of the readings or the condition of the patient". So in the usual case of prior art electronic glucose metering system the glucose meter device itself has a man-machine interface which has to be used by the patient in order to select the correct calibration curve corresponding to any respective test strip in use. However, due to the fact that the man-machine interface of the prior art metering system is very poor, as it has only limited input controls, the selection of a correct calibration curve is a very difficult task even for people who are quite computer-minded, and it is much more difficult to do it correctly for diabetes patients, who are less computer-minded, or who are in a typical "under-sugar" condition.

Also both before-mentioned German patent application documents do not provide any solution for facilitating the calibration task.

The before-mentioned MAJOR II electronic glucose meter system for example requires that the metering device must be coded manually to match a certain code number, which is printed on a respective blood glucose test strip vial. In particular, the user is required to insert a separate code card provided in the test strip package into a slot present on the meter device. Then the meter itself will beep, turn on automatically and will display a certain code number which must be checked by the patient to be identical with that one printed on the test strip package. A disadvantage of this system is that the handling of the code card implicates the risk that the sensitive test strips of the vial fall out of the vial, and are unusable in case they are moistened.

Further, the recognition of the code comprised of the code card works only, if the user hits the correct timing, when inserting the code card. Otherwise, the code is not read.

Further, the MAJOR II glucometer's time and date setting is either no implemented or difficult to do for the user, as it has only a single control button. So, in particular older, less computer-minded people will avoid such setting and consequently, any diary using the time information data of this prior art glucometer is not precise. Further, the display has no background light, thus a value can be hardly read in a dark situation.

But the most important disadvantage is that the MAJOR II metering device can not be used with test strips of a producer different to MAJOR II. Thus, in emergency situations, when no MAJOR II test strips are available the patient is not able to perform a precise glucose measurement with this glucometer device.

### 1.3. OBJECTIVES OF THE INVENTION

It is thus a general objective of the present invention to provide a more flexible and user-friendly method for processing measured values being related to a human body, in which method a property of a human substance is measured by a measuring instrument sensing characteristic changes of a test device effectuated when having contact with this human substance, and wherein one or more electrical quantities are obtained from said measurement.

It is a specific objective of the invention to provide a more flexible and user-friendly handling of a glucose meter system, and to provide a respective system therefore.

### 2. SUMMARY AND ADVANTAGES OF THE INVENTION

This objective of the invention is achieved by the features stated in enclosed independent claims. Further advantageous arrangements and embodiments of the invention are set forth in the respective subclaims. Reference should now be made to the appended claims.

The present invention provides a system and method for processing measured values being related to a human body, in which method a property of a human substance, i.e. blood, urine, saliva, etc., is analyzed. Special preferred embodiments are directed to the case that the measured values reflect the glucose level.

The basic idea of the invention includes the idea of removing most of input/output (I/O) capabilities and program tasks required for calibrating the measuring values, for example from a glucometer device and respective test strips in use, away from the metering device, and using instead the I/O capabilities like large display, explicit function keys and the CPU and storage resources which are already present at the separate, preferably mobile communication device, or, alternatively, being present at a Web Server being connectable to the user system.

By this general approach the required calibration of the measured values is significantly facilitated, as it may be automated easier by way of using the computational and the I/O resources present already on the communication device. Thus, for example a JAVA applet can be implemented at a mobile phone, which applet may run a workflow for either automatically calibrating the measured values in normal cases, and enabling manual, user-supported, or Web Server aided calibration in emergency situations when a first-choice, proprietary test strip is not available.

The basic subject matter of the independent method claims solve this general objective. The invention is "distributed" in nature over a plurality of devices, each of which has some contribution to it, and is programmed incorporating more or less decisive parts of the inventional method. The inventional method can be applied for measuring and processing measured values of blood pressure, of pulse, of blood or urinal glucose level, of adipose values, of gout-indicating blood values (uric acid), and in general, basically for any measurement the immediate result of which needs to be calibrated, in order to be able to interpreted by general medical knowledge, and independently of the measurement instrument in use.

A preferred embodiment of the system comprises at its user end two "pieces" of closely cooperating hardware, of which a first is a mobile communication device, like a mobile cellular phone, which is enabled to be programmed for running an additional application, e.g., by JAVA programming, and the second is the actual metering device, also referred to herein as measuring instrument, or glucometer device, as it is basically published in the before mentioned patent applications. Both devices comprise send/receive functionality provided in respective units in order to exchange digital information. Preferred is a wireless communication, like Blue-Tooth, IR, etc., but cable-based alternatives may also be implemented as they need no protection against exterior disturbance, in order to provide a cheaper interface without a sender and receiver circuit, antennae, etc., and without encryption, decryption, and user authentication software modules otherwise required.

The inventional approach is able to be applied for any type of sensor device, be that sensor located external of the human body, or externally affixed to it, or be that sensor implanted within the body, in order to allow for permanent, easy surveillance. This is relevant or the emerging market of such implantable sensors. The subcutaneous applications like subcutaneous glucometers are feasible, as the inventional method integrates a wireless connection between the subcutaneous measuring instrument and a further communication device.

Of course, instead of a mobile phone, also a Personal Digital Assistant (PDA) having cellular communication capabilities referred to herein as "Smartphone" can be used. In order to improve clarity, the term "Smartphone" is meant herein generally to include in particular a simple, but programmable, and in so far a little bit "smart", mobile phone.

So, basically, the present invention is distributed over several hardware devices and comprises functional, method-related as well as structural, device-related inventional features.

According to a first basic method-related aspect of the invention a method is disclosed - implantable for example as a Java Applet on a separate electronic communication device, in particular on a mobile phone - for processing measured values being related to a human body, in which method a property of a human substance, i.e. blood, urine, saliva, etc., is measured by a measuring instrument sensing characteristic changes of a test device, e.g. a test strip as known from glucose measurements, wherein the changes are effectuated when the test device has contact to the human substance, and wherein one or more electrical quantities, voltage, current, capacity are obtained from the measurement, which are converted into digital measured values,
wherein the method is alternatively done in three different variants A, B, and C:

In variant A the calibration is done locally at the measurement device, wherein the calibrating information is sent to this device from a separate communication partner device, preferably the above mentioned mobile phone.

In variant B the calibration is done at the communication partner device after this device has received the measured values. The calibrated values can then be output either on both devices or selectively at a single one of them.

In variant C includes "web calibration". Here, the calibration is done under inclusion of a calibration server, after issuing a respective request by and receiving the calibrated value at the partner communication device. Any suited protocol e.g., TCP, GSM, GPRS, SMS, FTP, etc. can be used to do that.

The method of variant A is thus characterized by - using a functionality-reduced measuring instrument comprising a sensor unit and a local (near field) communication unit, preferably a wireless send/receive unit including user authentication and encryption and decryption facilities, and an electronic communication device, which receives an ID of calibrating information for the test device (41) used during the measurement,
- providing a selection interface remote from the measurement instrument in the separate electronic communication device, preferably in a mobile phone or equivalent cellular communication based device like a smartphone, as mentioned further above, communicating also with the measuring instrument, for selecting a particular calibrating information out of a plurality of preferably pre-stored, and/ or down-loadable, test device specific information, e.g. calibration curves or respective unique test device IDs of a plurality of commercially available test devices, by using said received calibrating information, in order to at least prepare the calibration of the test device in use remote from the test device,
- sending the selected information to the measuring instrument in order to calibrate the measured property.

The method of variant B is thus characterized by receiving at the electronic communication device one or more measured values from the measuring instrument,
using the selection interface at the electronic communication device for selecting a particular calibrating information out of a plurality of pre-stored test device specific calibrating information,
transforming the one or more measured values into a calibrated value by a calculation performed at the communication device under inclusion of the selected calibrating information.

The method of variant C is thus characterized by
- receiving at the electronic communication device one or more measured values from the measuring instrument,
- forwarding the measured values and an ID of the test device used for the measurement to a predetermined remote calibration Server system usable for performing the calibration of said measured values, and
- receiving a calibrated value from the Server system.

After receipt the calibrated value is output to the user.

Thus, according to these three aspects the calibration procedure may be run with user-selected or at least user-triggered input data either
a) at the measuring instrument, or
b) at the Smartphone, or
c) at a Web server medicine station.

Input data from user selection in the above sense may be a calibration curve ID, which is displayed by the Smartphone as a member of a respective list and is enabled to be marked by the user and confirmed by him for appliance. Then, the calibration is done either at the testing device, after sending the calibrating information derived from the user input to the testing device, or calibration is done at the Smartphone, after the measured value was sent yet uncalibrated from the testing device to the Smartphone. In this case, only the Smartphone displays the Glucose value, and the metering device may additionally display the measured electric value in order to present further additional information to the user from which an estimation of the glucose level may be derived at least by a smart user. In the last case calibration is done at the Web Server, after the uncalibrated value was transferred from the metering device to the Smartphone and was forwarded including the input data preferably including the test strip ID from there automatically to the Web server.

Further, the inventional method can further be enriched by applying optional additional features as follows: When for example the method additionally comprises the steps of:
a) using a PDA as a selection interface for selecting the test device specific calibrating information, and
b) sending the calibrating information, e.g. a pre-selected calibration curve ID from a PDA device to the measuring instrument,
c) storing the calibrating information at the measuring instrument,
d) performing a complete calibration of the test device at the measuring instrument, and
e) displaying calibrated measurement values at the measuring instrument,
then the advantage results that, once the calibrating information is stored at the measuring instrument for a complete test strip vial, a glucose value is presented to the user also in cases, when the Smartphone is switched off, e.g. in an aircraft, or if the power supply thereof is interrupted.

When the separate communication device comprises mobile phone functionality, then the possibility is introduced to use a dedicated Server computer to perform the calibration and to interpret the measured value correctly. Then the user can be supplied with a message (e.g. SMS) telling him the glucose value resulting from the current measurement, maybe accompanied by additional medical information being input by a medicine involved.

When the measured values are evaluated electronically remote from the measurement device, e.g., in a PDA device operatively coupled to said measuring device, then the measuring device can be hold simple in technical structure, the users need not learn to use and control a further electronic device except their PDA assumed to be already in use.

Preferably the measured values are blood sugar or urine sugar values.

A further advantage of the inventional method is the so-called "auto-select" feature, which will be appreciated to be very useful in an emergency situation wherein the patient runs out of test strips and is depending on a third person's or a third producer's test strips not explicitly compliant to his own glucose meter device in use. Then, by taking profit of the invention a diabetes patient may use a glucose test strip of such foreign identity as long as he is able to know about the producer, and the test strip ID. Once this information is present, it may be input into the mobile communication device, using the inventional method. Then, either the calibrating information stored locally at the patient's mobile communication device is selectable locally and usable for calibrating the measured values, or otherwise, if not locally present, the calibrating information is fully automatically requested by a request issued via a mobile communication link from a calibration server forming part of the inventional system in an extended form. These requests can be preferably Web service requests, communicating with a Web Server via a wireless communication protocol, e.g. TCP, GPRS, UMTS, SMS, etc.. This Web Server is always held up-to-date, to collect continuously all relevant calibrating data for al test strips which are commercially available. Thus, after receiving the calibrating information from the calibration server, the measured values are automatically calibrated correctly.

This inventional "autoselect" feature, wherein the correct calibrating information is automatically provided via wireless communication at the diabetic user may be exploited and
combined with test strips having universal adapters to the electric interface in any proprietary glucose measurement device commercially available in the market. Thus, in above emergency situations a valid measurement can be performed with basically non-compliant measuring equipment.

Further, in case the calibrating curve is printed on the package of the test strip, the patient may compare this curve to a number of curves prestored on his Smartphone and may select the best fitting curve for calibrating the measured value. This is due to the fact that calibrating curves can be displayed in a standardized format at the test strip packaging or at the Smartphone display.

In addition, the printed calibrating curve from the test strip package can be scanned by a digital camera provided already at the Smartphone and can be vectorized by a dedicated applet of the Smartphone in order to create and use the correct calibration curve, in case the test strip calibration curve is not yet stored in the Smartphone.

### 3. BRIEF DESCRIPTION OF THE DRAWINGS

The present invention is illustrated by way of example and is not limited by the shape of the figures of the drawings. Unless explicitly differently stated, all drawings are schematic representations in which:
- Figure 1: illustrates the structural components of a prior art smart phone,
- Figure 2: illustrates the structural and functional components of an inventional smartphone used to implement an essential part of the inventional method,
- Figure 3: illustrates the structural and functional components of a glucose metering device which is functionally- reduced according to a preferred embodiment of the present invention,
- Figure 4: illustrates components of the external part of the inventional system of figure 2, which may be introduced into the inventional method according to a preferred embodiment thereof,
- Figure 5A and Figur 5B: illustrate major steps of the control flow during the inventional method in a preferred embodiment thereof, between a smartphone applet and a glucometer device applet (figure 5A) and between smartphone and diagnostic web server, respectively (figure 5B),
- Figure 6: illustrates essential data of a data set exchanged between smartphone and glucose metering device or web server, respectively.
- Figure 7: illustrates a typical measurement curve, here exemplarily an electrical current of a blood glucose measuring sensor in arbitrary current units over time in seconds.

### 4. DETAILED DESCRIPTION OF THE PREFERRED EMBODIMENT

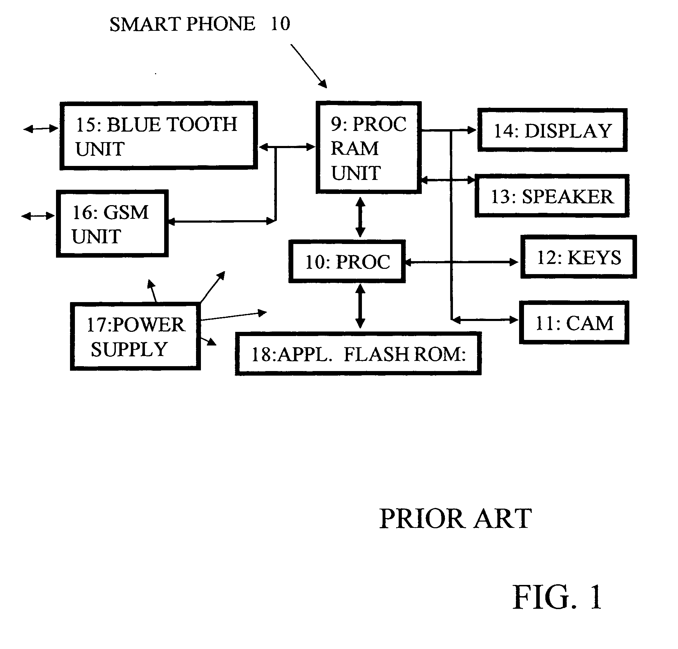

With general reference to the figures and with special reference now to figure 1 a prior art smartphone 20 has sufficient computing resources in form of a processor 19, an associated processor RAM-unit 9 loading and running the inventional Java Applet, and a Flash ROM 18, in order to run multiple program applications. Specific for a prior art smartphone 20 is the extended plurality of I/O capabilities in form of a camera 11, a keyboard with a plurality of keys 12 including alphabetic character keys 12, a speaker 13 and a large display 14. Further, a Blue Tooth unit 15 is provided as well as a GSM unit 16 in order to enable a communication in the near or the far environment, respectively. A power supply 17 is also provided in order to enable an operation of months /years without being connected to the mains.

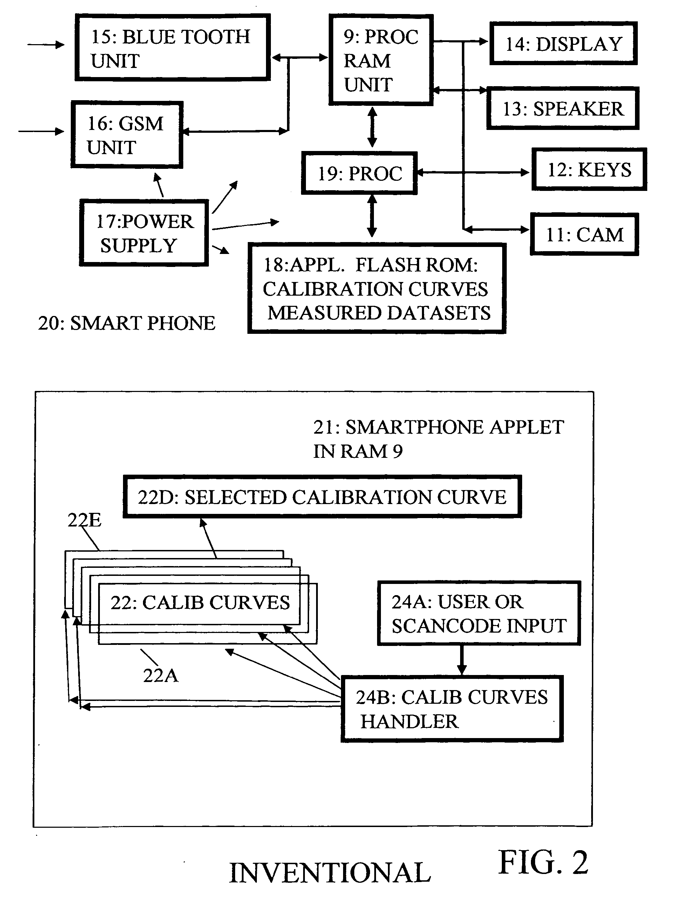

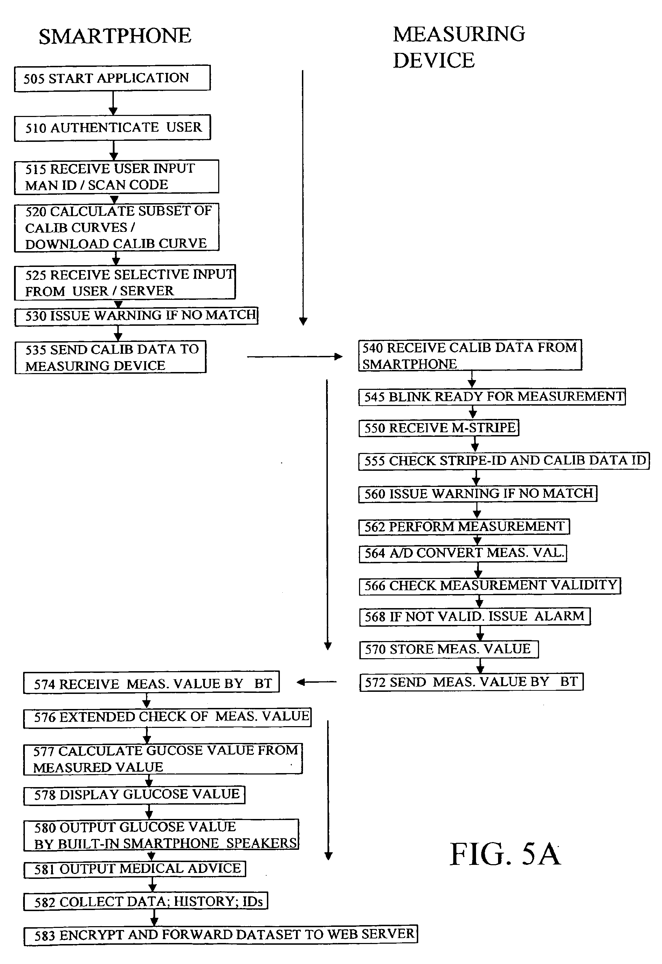

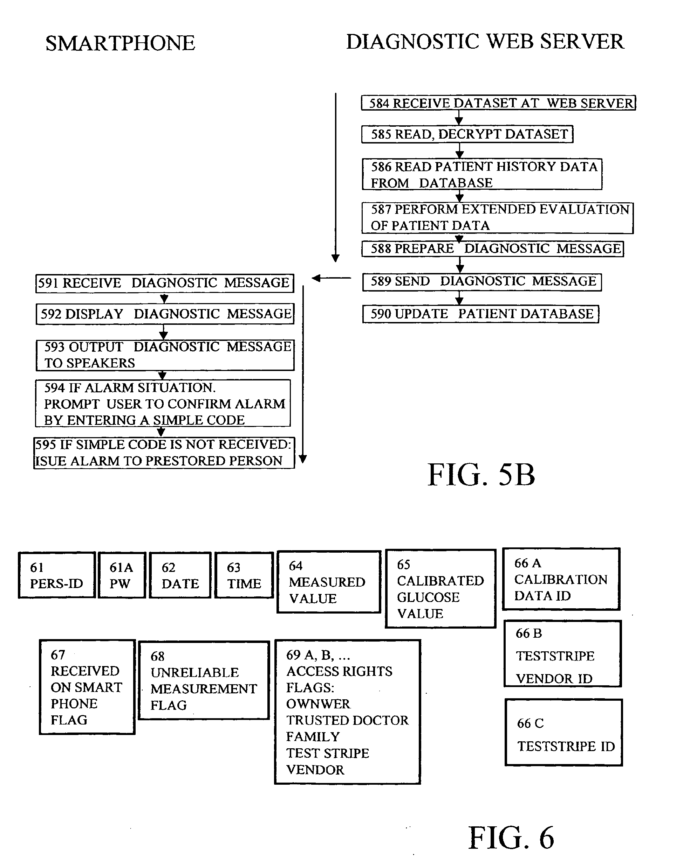

With further reference to figure 2 according to a preferred embodiment of the present invention a smartphone 20 comprises the above-described components 11, 12, 13, 14, 15, 16, 17, 18, 19 and 9. In so far, the description of those elements is done just by reference to figure 1. With reference to the present invention the RAM unit 18 of the smartphone is loaded with the before-mentioned JAVA applet 21, which implements most steps of the inventional method as they will be described in more detail in figure 5A, figure 5B and figure 6. In advance, and in short words the JAVA applet comprises a functional component 24 referred to herein as calibration curves handler which displays a list of calibration curve names 22A... 22E, which displays graphically the respective curves, when a user highlights one of the displayed calibration curve names by clicking a respective calibration curve name on the list, and which sends the respective data basically in form of a data array to a glucose metering device via its Blue Tooth unit 15.

Depending on the degree of customisation or the degree of user-friendliness smartphone 20 stores a more or less large plurality of calibration curves corresponding to a respective number of glucose test strip vials which are currently commercially available. In a quite extended form the plurality comprises several hundred of such calibration curves i.e., calibrating data, including calibrating data of basically all test strip producers worldwide, wherein each curve is accompanied and identified by a unique ID.

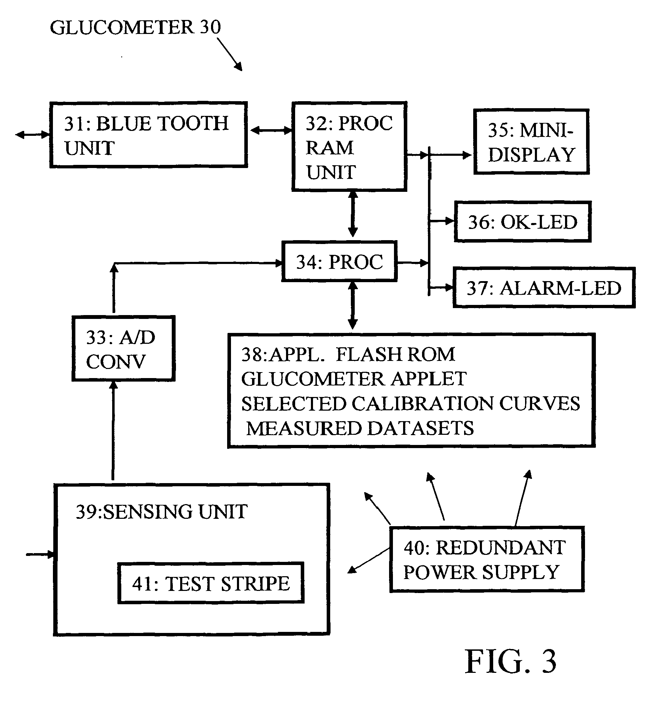

With reference to figure 3 a glucometer device 30 is described in more detail next below. The glucometer according to this preferred embodiment has a strongly reduced functionality implemented therein. In more detail a Bluetooth unit 31 is implemented in order to enable for a communication with the before-described smartphone 20. A processor 34, a processor RAM unit 32 and a Flash storage unit 38 is provided in order to implement only a limited plurality of functions further described below. A small display 35, an OK-LED 36 and an ALARM-LED 37 are provided in order to signalize only the most important information to the diabetic user. A prior art sensing unit 39 includes all necessary mechanic elements in order to receive a prior art glucose test strip 41 and to analyse the blood thereon according to prior art methods. A redundant power supply 40 is provided in order to guaranty operation of the device even if one pair of batteries is exhausted. An Analog-Digital (A/D) converter 33 converts an electrical property resulting from prior art sensing unit 39 and being representative for the proportion of glucose in the blood sample on the test strip.

It should be noted that the inventional principle is independent of what exactly is the electrical property or the physical property measured within unit 39. In most cases an electrical voltage is measured and converted into a digital value which is then further processed by the glucose meter applet provided in the application RAM 32.

As will be further be described in figure 5A and 5B the glucometer applet accesses the Flash ROM storage 38 in order to get the appropriate calibrating data curve stored there, and convert the digital value after A/D conversion into the correct glucose level value in order to be displayed on the mini display 35 for enabling the user to appreciate its situation and possibly apply the correct dose of insulin. Thus, an easy-to-use calibration is obtained by the invention.

A data set comprising essential information about the glucose measurement is then sent via the Bluetooth interface 31 to the smartphone 20 of the patient, where it is received by the respective Bluetooth unit 15, and displayed to the user. Details of this data set are further described with reference to figure 6 later below.

According to a specific further aspect of the invention this data set is preferably automatically sent via the smart phone's GSM unit 16 to an appropriate, centralized server where this data can further be processed.

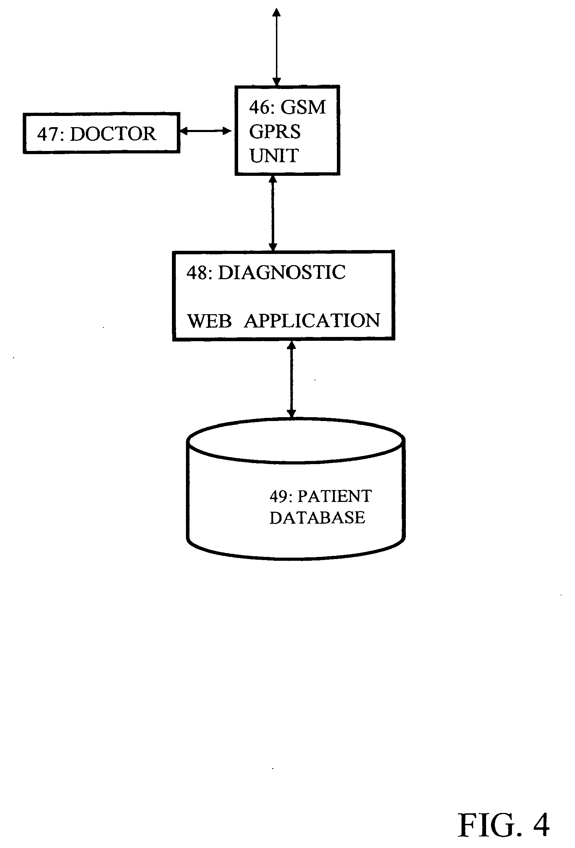

Figure 4 illustrates roughly the structure of such server. The GSM unit 16 of the smartphone comprises in this preferred embodiment two different telephone numbers, one of the personal doctor of the assumed patient, and the other of the before-mentioned GSM server. In this preferred embodiment GPRS packets are sent via GSM which are received by either the doctor's GSM unit 47 and/ or the GPRS unit 46 of the centralized server. It should be noted that messages according to other protocols can also be sent instead, e.g., TCP, SMS, UMTS. The server essentially comprises a diagnostic application 48 operatively connected to a patient data base 49. Many details of the services rendered by the diagnostic application 48 are disclosed in above-mentioned international patent application WO 2004/027676, see for example its figure 2, 3, 4 and 5 including the description thereof in this prior art document. So, the glucose value can be assessed by either a human person skilled in the medical art, and by automatic, program controlled medical services, which of course are not assumed to be able to replace a true medical advice given by a doctor, but which may serve at least as a useful diagnostic hint, when the patient's historic data stored in the database are involved in this "automatic" medical service.

With further reference to figures 5A and 5B details of the control flow in the communication between smartphone 20 and measuring device 30, as well as the communication between smartphone 20 and diagnostic server 48 are described in more detail.

In this sample workflow a diabetic user of the inventional method is assumed to perform a glucose value measurement with a test strip, a smartphone 20 as described in figure 2 and a function-reduced glucometer device 30 as described with reference to figure 3.

In a first step 505 the user starts the JAVA applet on his smartphone by pressing a key which has been dedicated to this function, before.

In a normal operation mode the JAVA applet asks for a user ID and a password to be input and confirmed by the user. This authentication procedure of step 510 is preferably demanded in case the user wants to forward the measured glucose value to a central database, where his personal data is stored. In emergency mode the user authentication may be omitted, in order to avoid complications.

Then the user checks the test strip or the test strip vial for occurrence of a test strip ID, a scan code etc., which may be adapted to identify the physical properties of the current test strip. In case there is a scan code, the user may take a photograph of the scan code by means of a camera provided by the smart phone, or alternatively, may input manually the test strip ID, step 515, or see the user input scan code input 24A in figure 2.

In most cases, an alphabetic code of six digits should suffice especially in cases, where the JAVA application was told about the test strip producer firm, in order to provide a sub-selection of available test strip IDs, by excluding non-relevant test strip IDs of third producers, of which the diabetic user processes no test strips.

A further alternative for the selection interface 24 is that the user is displayed a list of pre-stored test strip IDs, and he selects one from the list. The JAVA applet allocates a certain memory area in the Flash RAM 18, in which for each test strip ID calibrating data is pre-stored. Thus, by indicating the test strip ID, the JAVA applet is provided with appropriate calibrating information for a respective test strip.

In a next step 520 the smartphone JAVA applet 21 checks the user input or the scan code input 24A for the test strip ID and selects the one single calibrating curve 22D by performing a cross reference in a respective table "calibrating curve ID /calibrating data", stored in the smart phone's application Flash RAM 18, see back to figure 2. This function is implemented by the so-called calibration curves handler 24B, as depicted in figure 2. In case there is no calibrating data stored for a certain test strip ID the smartphone 20 uses automatically its GSM unit 16 in order to issue a GPRS call to the pre-programmed dial number associated with the GPRS server described with reference to figure 4, in order to download the correct calibrating data for this particular "strange", unknown test strip.

For this purpose, the diagnostic application 48 (see figure 4) provides a test strip calibrating data collection basically from all test strip producers. Then, the web application 48 checks the test strip ID input by the user and performs a cross-check in this data collection which ends up in selecting the appropriate calibrating data. Then it sends this calibrating data back to the requesting diabetic user. In the end of step 520 in most cases there will be a selection for the correct calibrating data completed and the calibrating data present either pre-stored in the Flash RAM of the smartphone 20 or freshly received via the GSM unit 16 of the smartphone 20.

In a next step 525 this "selective input" either from the user or from the server 48 is received in the applet 21 logic. In step 530 a warning is issued in those -presumably rare- cases in which there is no match between the demanded test strip ID and available respective calibrating data.

In this exceptional situation an exception processing will be triggered, which may enable the diabetic user to select "the best possible" calibration curve as follows:
this exceptional processing assumes that the calibration curve of the test strip is graphically depicted on the test strip vial in a standardized form, i.e. a x-y-graphic with standardised x, y-scalings. The user will again take the smart phone's camera and make a snapshot of the curve. The JAVA applet 21 scans the curve from the photograph and vectorizes it in order to determine a table of x, y-coordinates corresponding to the calibration curve. Then, by using a prior art best match algorithm the smartphone applet 21 compares its pre-selected calibration curves to the scanned calibration curve and determines the best match pre-stored calibration curve. This pre-stored calibration curve is then selected for further use.

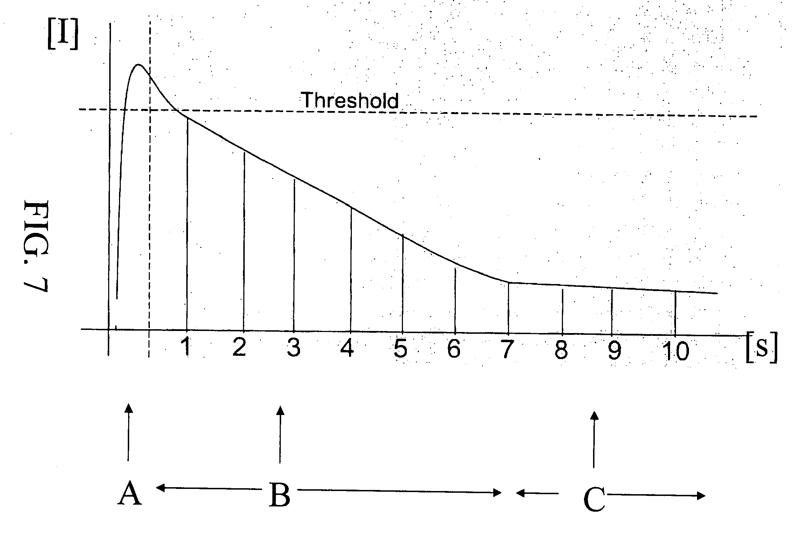

It should be noted that the best match algorithm used in here can be adapted to weight certain regions of the calibration curve as one is depicted and discussed exemplarily with reference to figure 7 - for example the position of a sharp peak - higher than other regions, which are less significant. By that, the particularities of glucose test strip calibration curves can be adopted selectively.

In step 535 the calibrating data which was determined during the preceding steps, is sent via the Bluetooth unit 15 to the measuring device 30, which in turn should be in the near Bluetooth environment, in order to receive this data.

In the next block of steps the workflow performed by application 38 implemented in the glucometer device 30 is described in more detail:
In step 540 the calibrating data belonging to the test strip just mentioned before is received from the smartphone at the glucometer device 30 via its Bluetooth unit 31. The data is processed by application 38 by means of built-in processor 34 and processor environment 32 (processor RAM). Then an optional check is run regarding the validity of the received calibrating data. After successful validity check the glucometer device 30 issues a "ready for measurement"-signal, for example a blinking of the OK-LED 36 in step 545 in order to prompt the user to perform the measurement. The user can now be assumed to provide the blood sample by applying some quantity of its own blood to the sensor region on the test strip of which the calibrating data is present in the glucometer device 30.
In step 550 the user puts in the blood-filled test strip, and the glucometer device receives the strip within a respective opening provided therefore: When the test strip is completely input into the strip opening the measurement begins and is performed according to prior art. In step 555 the sensing unit 39 verifies if the strip ID and the calibrating data ID just received before do match or not. In order to do that, the strip ID is read by a respective code reading device (scan code or binary code). If they do not match the glucometer device applet 38 issues a warning by displaying a respective warning message on mini display 35 and by blinking the alarm-LED 37.
In step 562 the actual glucose measurement is performed according to prior art measuring methods. It should be noted that the inventional principle is adaptable to incorporate any prior art measuring methods, which need calibration. The measurement is initiated and run automatically without any interaction of the patient to be required.

Assume, the measurement includes the sensing of a plurality of ten value pairs, of which one component is a time and a second component is an electrical value, for example an electrical voltage.

In step 564 all those measured values are converted from analogue to digital within A/D-converter 33. With particular respect to the typical glucose measurements in prior art a typical measured curve shows a relatively sharp peak, followed by some form of decay curve. Thus, the peak is sampled as well as possible, and the most significant points of the decay are also collected. The sample rate is preferably adjusted such that sharp increases or decreases are sampled with higher density than regions of the curves which show a more or less flat behaviour.

In step 566 the measured values are subjected to a validity check, which includes to issue a warning if the measured values seem to be not plausible, or at least if they do not lie in a value range, which is conceivable as "possible in reality".

If the measured value is plausible then the OK-LED 36, preferably green-coloured is activated, otherwise in cases of non-plausibility the alarm-LED 37 is activated, preferably red-coloured.

So, in step 568 an alarm is issued if the measured values are not plausible. The alarm may be issued via the mini display 35 and by means of the alarm-LED 37.

In step 570 the measured values are stored in the Flash RAM of application 38 in a data set of a given, predetermined data format. This format includes the glucose value as it results from calibration and - amongst others optionally - also the electrical value or a plurality of values from which the calibrated glucose value was generated.

Then, in a step 572 the glucometer applet 38 sends the data set automatically to the smartphone 20.

In the next sequence of steps the JAVA applet 18 implemented in the smartphone 20 becomes active again: in a first step 574 the applet 21 receives the data set including the glucose value by its Bluetooth interface 15.

After good receipt of the data set the smartphone acknowledges the receipt of the data back to the glucometer device, which switches back into an energy-saving stand-by mode after having received this acknowledgement. It should be noted that the Bluetooth transmission should be dimensioned such that a distance of three meter should be within the range of the transmission.

The overall duration of the Bluetooth transmission required for the data set should be as small as possible in order to save energy and keep possible interferences with other Bluetooth transmissions in the immediate environment of the patient's Bluetooth sender as small as possible.

As such glucose values are basically of very personal nature it may be required to encrypt all data before being sent. In this case an appropriate state-of-the-art encryption/decryption method should be used with a key having a sufficiently long length. Further, advantageously the measured data is buffered in the Flash RAM 38 of the glucometer device in order to be able to re-use the data for repeated sending to the smartphone in case the smartphone is temporarily not available via the Bluetooth connection. So, the glucometer device preferably sets a flag indicating for each data set if or if not the smartphone 20 has already acknowledged receipt of the data set. In case the smartphone is again reachable via Bluetooth the glucometer device sends all measured data sets which are indicated by the flag as "not yet successfully transferred".

In a step 576, optionally, further plausibility checks are performed, in step 577 the glucose value is calculated from the measured value by involving the calibrating information, for example the value pairs from the calibration curves. Then, further optionally, for different glucose value ranges, respective different voice patterns "accompanying the glucose value" are stored in the application ROM. One of it is associated with the calculated glucose level value for being output after conversion into the glucose value.

In step 578 the glucose value is displayed at the Smartphone display 14.

In a next step 580 the smartphone 20 outputs the glucose value by its built-in smartphone speakers 13. Such speaker output could be something like: "Your glucose value is momentarily at a value of... [glucose value]". This is a feature which is particularly advantageous for older people or for people having some deficiencies in recognising optically displayed, small details.

Further, the accompanying voice patterns mentioned above can be output. For example, "You should visit immediately a doctor", in case the value is extremely high, or "You should eat two pieces of chocolate", if the value is in a moderate range lower than the range, usually understood as normal.

This feature can be extended to be personalized, in order to be adapted to personal particularities, like body weight, age, etc..

Additionally, voice message might be extended to be proactive in nature, when a personal activity/time schedule has once been input into the mobile phone, if it is foreseeable that the patient will undertake some physical efforts, by doing some sports, etc., in the near future. This can be implemented just by doing a cross reference into the scheduler of the mobile phone and reading the activity of the next few hours.

Then in a next step 581 the applet 21 implemented in the smartphone 20 optionally outputs a medical advice again via display 14 and speakers 13.

In the next step 582 the smartphone application collects all measured data not yet sent to the above-described diagnostic application 48 (figure 4), performs an encryption of the collected data in a step 583 and forwards the encrypted data set to the diagnostic application 48. As mentioned before, this is done preferably by sending data packets according to the GPRS. It should be noted that the data is encrypted again as appropriate before being sent to server 48.

The next steps are performed at the diagnostic server 48:

In a first step 584 the server application 48 receives the data set. It then reads and decrypts the data set in a step 585.

Then, in a further step 586 it accesses the patient database 40 and collects history data already stored in this database for this specific diabetic user. Then in a step 587 an extended evaluation of the patient data is done. This preferably begins with a filter algorithm which filters out all those cases in which the glucose value lies within a personal glucose range which can be assumed as to be tolerable for the actual patient and not to be dangerous for his health. Preferably, only those cases are processed in more detail in which the current glucose value is beyond the limits of such tolerable interval. The setting of the interval should be done carefully, preferably patient-specific and involving the patient's doctor.

In this case beyond those limits the diagnostic application automatically calls the doctor 47 of whom the address and telephone number is stored as a basic data within the patients data set.

In step 588 so, the youngest, historic data of the patient stored in the database for the patient is collected, in order to know about the last changes of the glucose level of the patient, and this data collection is sent to the doctor also preferably using the GPRS service. Then, always as part of the diagnostic message preparation step 588 the doctor's answer is expected containing a personal diagnostic message, for example indicating the recommended quantity of insulin to be applied to the patient. After a certain predetermined time-out has passed without any answer message of the doctor being received by the diagnostic application 48, the application 48 prepares its own diagnostic message by using pre-stored diagnostic recommendations, also stored within the patient's database, and this diagnostic message is then sent to the patient's smartphone 20 in a step 589. The actual diagnostic messages are preferably designed such that they must have been input into the personal database by the doctor, before.

Then, in a step 590 the patient's database is updated with the current glucose level and the current time and date of the last measurement.

The next steps are again performed at the smartphone of the patient:

The smartphone 20 receives the diagnostic message - either assembled by the doctor or automatically generated by the diagnostic application 48 in step 591. In a next step 592 this diagnostic message is displayed at the display 14 of the smartphone 20 and is preferably concurrently output by a human voice to the speakers 13 of the smart phone, step 593. In absence of this human voice audio output, the speaker can be replaced by an electronic output means and may be reduced to two or three different signal types, for example an alarm beep, a "moderate pre-alarm" beep and an OK-beep.

Then, according to a special inventional, add-on feature, in a next step 594, if this is an alarm situation, the user is prompted by the smartphone to confirm the alarm by entering a simple, predetermined alarm code, in order to check, if the patient possesses his full consciousness. In cases of full consciousness it can be assumed that no emergency case is present. Otherwise, after a certain predetermined time-out of about one minute, which is filled with further prompts to input the alarm code the smartphone 20 will issue an alarm message to a person (GSM), which is pre-stored in the smartphone as a personal assistant for emergency cases. By this additional feature it is possible to issue an alarm only in the very rare cases of real emergencies, step 595.

With further reference to figure 6 the elements of a data set as they appear relevant for the purpose of the present invention and as prepared by the inventional system devices and sent from the glucometer device 30 to the smartphone device 20 are depicted and will be described in more detail next below:

First, a personal ID 61 is needed in order to clearly identify the diabetic patient in question. An example could be name and private address of the patient. In field 61A an encrypted password is provided. This data is input into the smartphone 20.

Next, in fields 62 and 63 date and time of the glucose measurement is stored. This data is generated basically by the glucometer device 30 in the very moment when the glucose measurement is actually done. In order to avoid time and date errors the smartphone makes a plausibility check on any time related data as it is provided with an own timer. In case of ambiguities, say time indications differ by more than ten minutes, a flag 68 is set indicating that the measurement is unreliable. Of course, alternatively, a special flag indicating that only the time information is not reliable may be generated and stored separately for example in a separate field.

Then, in data field 65 the calculated glucose value is stored as it is calculated by the glucometer application 38 including the calibrating information. Additionally, in field 66A the calibrating data ID as determined by the smartphone 20 is stored, in field 66B the test strip vendor ID, i.e, for example the name of the vendor enterprise is stored, and in field 66C the (mostly) vendor-specific test strip ID itself is stored.

With these two pieces of information the calculated glucose value can be approved and verified later, after measurement, either by the smartphone application or by the diagnostic server application 48, or by the doctor 47.

Then, in field 67 a flag is stored by the glucometer device indicating if or if not the data set was completely received at the smart phone, as it was described further above.

In field 68 a flag can be set indicating that the measurement is not reliable due to physical deficiencies of the test strip as it was described further above, for example due to impurities or moisture of the test strip. This data is determined by the glucometer device if respective moisture or impurity sensors are provided thereon, or is manually input by the user via the smartphone's input means.

Further, in field 69, A, B, C, ... different access right flags can be set in order to allow or not allow to read the data set or parts thereof. One may differentiate groups as follows: owner, trusted doctor, family member, test strip vendor, clinician, medical administrator of patient database 49, etc. Respective group names are stored also.

It should be understood that field 64 comprising a single or a plurality of measured electrical values in a digitalised form is optional. The electrical values may help in some rare cases in which the calibrating information is not reliable and in which the user has some knowledge about the electric values from measurements taken in the past. But it may also confuse other persons not being so familiar with the measurements.

In variation to the first embodiment just described above the glucometer device 30 according to a second embodiment is calibrated by a respective web service instead of being calibrated by calibrating data input manually by the user. The only technical precondition to do, is to have a worldwide valid system for uniquely identifying test strips and to store the calibrating information in a centralised form and associated with and accessible by this test strip ID. This web-based calibration relies on the fact that the web server can be reached by the smartphone using a communication path as currently available, be that by mobile phone and GPRS, which is preferred, or by SMS, or UMTS, or be that by establishing an Internet connection between the smartphone and the diagnostic application 48 depicted in figure 4 via UMTS or a classical, cable bound web link.

In all other situations, in which an offline capability is required, the smartphone stores a respective plurality of pairs: test strip ID and memory address of respective calibrating information in the Flash RAM of the smart phone. Also, the features of both embodiments can be combined for a third embodiment.

In all those embodiments, the diagnostic web application 48, when being in contact with a smartphone 20 application 21 via web requests or other connections, will advantageously check the time stamp of the software 21 in use at the smartphone and that of the glucometer. Both software products are updated preferably automatically via such link. This includes also the updates of calibrating information for newly fabricated test strips of different test strip vendors.

Next, and with reference to figure 7 a typical measurement curve, here exemplarily an electrical current of a blood glucose measuring sensor in arbitrary electrical current units over time in seconds, is depicted for a glucometer device 30.

In this measurement a small quantity - a not too small drop - is taken from the human body, and is applied onto the sensible area of a test strip. The sensor is activated and senses the physiological property it is dedicated for. In this example this is the conductivity of the blood. The conductivity is measured at a plurality of times 1.. 10.

By bringing the sensible area of the test strip 41 and the blood into contact with each other, a chemical reaction is triggered, by which reaction the blood changes its electrical resistance, and thus conductivity dependent of the glucose level present in the blood sample.

At the beginning, see initialization phase A in figure 7, the conductivity, and thus the sensing electrical current must be beyond, i.e. greater than a predetermined trigger level, indicating a valid measurement. If this level is not reached the measurement is aborted, in order to exclude cases, where the blood is no more fresh enough, or the measuring device 30 or test strip 41 is damaged anyway.

In case the measurement is assessed valid, a number of ten measurements of electrical current I are sampled, and the glucose level is calculated according to prior art under inclusion of the calibrating information associated with an individual test strip or test strip vial. A person skilled in the art will appreciate that different strips from different producers will produce different curves having different amplitudes and decay behaviour, especially in phase B, and furthermore in phase C. This is the reason, why the calibration of the measured values is a highly sensitive and thus important task in order to generate and output a reliable glucose level.

According to a further aspect of the present invention the diagnostic application 48 also has an Internet interface, as basically described in above-mentioned patent application DE 10140968A1. This implies the advantage that the patient may have a look on his personal data collection via the Internet and a trusted https-access to its personal account on the patient database 40. In order to do that, an appropriate web server application is provided. Also a doctor having access rights to the patient's database may take a closer look on the patients data history and has thus an easy-to-do and complex view on the patient's personal health data which enables him to provide the patient with a quite founded remote-diagnosis using just the mobile phone link.

Further modifications and advantageous features of the method of the present invention are disclosed in order to further enrich the preferred embodiment just described before, or to enrich more basic and function-reduced variations thereof as they appear in the claims, and in particular in the more basic claims:

The JAVA applet 21 implemented on the smartphone 20 of the patient may be controlled by the patient to display more than the last measured value, e.g., the values of the last few days, or dependent of the storage capacity in use may display values of the preceding week or several weeks. Preferred is a diagram "glucose value over time".

Further, the applet 21 also offers a "diary function". In this diary the patient may append some text as a comment to each measured glucose value, in which text the patient may specify special events of his life. Such events may be physical or mental stress or periods of increased sugar consumption by eating sweets like cakes, chocolate, etc., an increased or decreased quantity of liquid consumption, alcohol consumption, etc.

The basic version of the glucometer device 30 has no separate keys for controlling the device, but instead only the slot for receiving the test strips, a small display, an OK-LED and a NOT-OK-(alarm) LED. Both LEDs may be used to tell the patient if the test strip is completely inserted, if the measurement was successful or not, or if the batteries should be changed.

The mini display mentioned above is useful if the connection to the smartphone is temporarily not available. The display should have some illumination. A slightly modified version of this basic functionality of the glucometer device 30 includes an acoustic output generator device, which may have at least the functionality of the both LEDs mentioned before, by issuing the typical acoustic signals for signalising OK or NOT-OK, as they are broadly accepted in the man-machine interfaces of prior art mobile phones, PDAs, etc., in order to give the user an acoustic feedback if a given control operation was successful or not.

So, the skilled reader will appreciate that the present invention provides several alternatives in order to free the diabetic user of the glucometer device from most of the burden dealing with correct or incorrect calibration of the test strips in use.

Further, by reducing the functionality of the glucometer device and by advantageously using the smartphone of the patient as the device for doing some selective input regarding the correct selection of the calibrating information, the user may concentrate to the correct control of a single electronic device, which is basically its smartphone, or PDA, etc. Thus, the user needs not learn to control two different electronic devices.

Further, the inventional concept also offers special solutions for older people who are in many cases less computer-minded than younger people. For those people the JAVA applet 21 may be activated by a preferred button or may even be activated automatically and the user can be automatically prompted to perform a new glucose level measurement. All other functionality is done automatically without any intervention by the user being required.

So the inventional concept is particularly useful for those non-computer-minded people.

As the method according to the invention provides for a high quality of the measured values due to improved calibration of the glucometer device the measured values are more reliable than in prior art. Further, the increased reliability is accompanied with very fast deliverage of the measured values to the personal doctor associated with the patient. This helps to provide immediate diagnostic or physical help to the patient, in particular in emergency situations if the glucose level is extremely high or extremely low.

Further, the JAVA applet 21 can also be implemented in a PDA or mobile phone already existing and in current use of the patient just by downloading the applet, installing it and customising the mobile phone in the right way for offering an easy-to-do activation, for example by reserving one control button for it or by moving the applet into a high priority position in the menu surface of the mobile phone.

The present invention can be realized in hardware, software, or a combination of hardware and software. A calibration tool according to the present invention can be realized in a centralized fashion in one computer system, or in a distributed fashion where different elements are spread across several interconnected computer systems. Any kind of computer system or other apparatus adapted for carrying out the methods described herein is suited. A typical combination of hardware and software could be a Smartphone computer system with a computer program that, when being loaded and executed, controls the computer system such that it carries out the methods described herein.

The present invention can also be embedded in a computer program product, which comprises all the features enabling the implementation of the methods described herein, and which - when loaded in a computer system - is able to carry out these methods.

Computer program means or computer program in the present context mean any expression, in any language, code or notation, of a set of instructions intended to cause a system having an information processing capability to perform a particular function either directly or after either or both of the following
a) conversion to another language, code or notation;
b) reproduction in a different material form.

## Claims

1. A method for processing measured values being related to a human body, in which method a property of a human substance is measured by a measuring device (30) sensing characteristic changes of a test device (41) effectuated when having contact with said human substance, wherein one or more electrical quantities are obtained from said measurement, which are converted into digital measured values, further evaluated at a communication device (20) communicating with said measurement device (30) **characterized by** the step of:
said communication device (20) making a plausibility check on time related data associated with a data set generated by said measurement device (30).

2. The method according to claim 1, wherein said separate communication device (20) further comprises mobile phone functionality.

3. The method according to claim 1 or 2, wherein from said measured values a physiological value of the group of:
a) blood glucose or
b) urine glucose
c) adipose,
d) gout,
e) blood pressure,
f) pulse
is derived.

4. A communication device (20) for use in communication with a measurement device (30) for processing measured values being related to a human body, in which method a property of a human substance is measured by said measuring device (30) sensing characteristic changes of a test device (41) effectuated when having contact with said human substance, wherein one or more electrical quantities are obtained from said measurement, which are converted into digital measured values, further evaluated at a communication device (20) communicating with said measurement device (30),
the communication device (20) being **characterized by** a programmed control component performing the step of:
making a plausibility check on time related data associated with a data set generated by said measurement device (30).
